# EUROPEAN PATENT APPLICATION

(11) **EP 2 823 847 A1**
(43) Date of publication of application: **14.01.2015**
(21) Application number: 13757386.1
(22) Date of filing: 04.03.2013
(51) Int. Cl.: A61M 25/10

(54) **BALLOON FOR IABP BALLOON CATHETER**

(30) Priority: 08.03.2012 JP 2012051664
(71) Applicant: Tokai Medical Products Inc., Aichi 486-0808 (JP)
(72) Inventor: TSUTSUI Nobumasa, Kasugai-shi Aichi 486-0808 (JP); TSUTSUI Yasuhiro, Kasugai-shi Aichi 486-0808 (JP); MURAKI Yasuhiro, Kasugai-shi Aichi 486-0808 (JP)
(74) Representative: Zeuner Summerer Stütz
(86) International application number: PCT/JP2013/055811
(87) International publication number: WO 2013/133201

(57) **Abstract**

The external surface of a balloon for IABP balloon catheter is designed to have a rough surface occupying an area of 10 to 80% both inclusive of the whole of the external surface, and a smooth surface occupying an area of 90 to 20% both inclusive thereof. The supplied balloon for IABP balloon catheter is certainly unwrapped by a predetermined balloon pressure from a driving machine, with no need to conduct any preloading process, and further which is not unwrapped before and during the insertion of the catheter even in the state that a wrapping sheath is removed.

## Description

### TECHNICAL FIELD

The present invention relates mainly to a balloon for IABP (Intra-Aortic Balloon Pumping) balloon catheter.

### BACKGROUND ART

IABP is a treatment technique of: inserting, at the time of a fall in cardiac function, such as cardiac insufficiency, an IABP balloon catheter into an aorta; and repeating the inflation and contraction of a balloon for IABP balloon catheter (hereinafter also referred merely to as a "balloon" hereinafter) that is located at the front end of the IABP balloon catheter while synchronizing the inflation and contraction with heartbeats, thereby increasing the stream of blood in the coronary artery and the whole body to assist the person's cardiac function. At the time of the treatment, this IABP balloon catheter is connected to a driving machine, and the driving machine is used to provide positive pressure and negative pressure inside the balloon to inflate and contract the balloon.

The balloon used for the IABP method, to increase the assisting effect, is fitted with a columnar balloon having a slightly smaller diameter than the inside diameter of the aorta. The diameter of recent IABP balloon catheters has been becoming smaller for decreasing the invasive effect thereof. The outside diameter of ordinary IABP balloon catheters is from about 2 to 3 mm. Accordingly, before their balloon is inserted, the balloon is very tightly wrapped. The period of use of any balloon catheter in the IABP method may be over one month or longer. Thus, for the IABP balloon catheter, polyurethane is generally used, which is high in compatibility with blood. However, polyurethane has a nature that when the external surface thereof is a mirror surface, regions of this material easily adhere to each other.

For this reason, after the insertion of the balloon, the adhesion of regions of the resin, which constitutes the balloon in the state of being wrapped, cannot be cancelled by the inflation thereof by use of only the driving machine. Thus, the balloon may be incompletely inflated. In order to prevent this incomplete inflation, the volume of air that is about 20 to 30% larger than the volume of the balloon is injected into the balloon through a syringe or the like, so that the inside of the balloon is made into the state of having a higher pressure than the inflating pressure attained by the driving machine for balloon-inflation. This manner cancels the adhesion of regions of the resin of the balloon to each other to inflate the balloon completely once. This preloading process is conducted prior to use of the balloon catheter. However, this process has a problem of requiring much time and effort in case of emergency.

Such a balloon is inserted into the body after a wrapping sheath thereon, which is fitted thereto for holding the wrapping for the balloon, is removed. However, there remains a problem that the insertion of the balloon will become difficult if the wrapping for the balloon of the IABP balloon catheter is unfavorably loosened prior to the insertion.

### CITATION LIST

### PATENT LITERATURE

Patent Literature 1: JP 2002-263193 A

### SUMMARY OF INVENTION

### SOLUTION TO PROBLEM

In order to attain the above-mentioned object, the present invention adopted the following means:

The balloon of the present invention for IABP balloon catheter has a structure in which the external surface of the balloon for IABP balloon catheter has: a rough surface occupying an area of 10 to 80% both inclusive of the whole of the external surface; and a smooth surface occupying an area of 90 to 20% both inclusive of the whole. According to this adopted structure, the region of 20% or more of the external surface of the balloon for IABP balloon catheter has the smooth surface; thus, even when the balloon is inserted into a blood vessel in the state that a wrapping sheath is removed, the balloon has an enough adhesive strength to prevent unwrapping of the balloon. For this reason, the balloon can be inserted into a target position (detention position) without being unwrapped. On the other hand, the balloon has the rough surface in the region of 10 to 80% both inclusive thereof, which makes it possible to decrease contact surfaces of the resin with each other to decrease the adhesive area and the adhesive strength. For this reason, without conducting any preloading process, the present invention makes it possible to produce a balloon that can be unwrapped only by inflation based on a predetermined pressure from a driving machine.

In the balloon of the present invention for IABP balloon catheter, the rough surface may be dispersed in at least the entire external surface other than a conic part located at the distal side or proximal side of the balloon for IABP balloon catheter. According to this adopted structure, the provided balloon can be a balloon having a substantially uniform adhesive strength and peelability, over the balloon external surface other than its conic part located at the distal side or proximal side of the balloon, to extend to regions from the distal side of the external surface to the proximal side thereof. In the present specification and the claims, the wording "distal side" denotes the inserting side of an IABP balloon catheter; and the wording "proximal side" denotes the operator's side of the IABP balloon catheter when its balloon is operated.

In the balloon for IABP balloon catheter according to the present invention, the rough surface may be formed only in a location other than the conic part formed at each of the distal side or proximal side of the balloon for IABP balloon catheter. In other words, the rough surface is formed only in a columnar part of the balloon, which is other than the conic part formed at the distal side or proximal side. When the rough surface is formed in the columnar part, the balloon in the columnar region is certainly peeled to be loosened. Since the contact area of the conic part is small when the balloon is wrapped, the conic part can easily be loosened. Consequently, without conducting any preloading process, this structure makes it possible to produce a balloon that can be unwrapped only by inflation based on a predetermined pressure from a driving machine.

In the balloon for IABP balloon catheter according to the present invention, the rough surface may be a crepe surface or a grain surface. With this structure, it is possible to provide a balloon for IABP balloon catheter that has an appropriate adhesive strength and peelability by adjusting the roughness of its crepe surface or grain surface.

In the balloon for IABP balloon catheter according to the present invention, the rough surface may be formed into any one of striped, checkered, dotted, and spiral patterns, or into a pattern of a combination thereof. The formation of any one of the various patterned rough surfaces makes it possible to select an optimal rough surface in accordance with the production method of the balloon. The formation also makes it possible to form a rough surface having regions dispersed averagely in the surface of the balloon. Thus, this structure makes it possible to provide a balloon having a uniform adhesive strength and peelability over the whole of the external surface.

In the balloon for IABP balloon catheter according to the present invention, the rough surface may be formed in at least one part of the internal surface of the balloon for IABP balloon catheter. With this structure, it is possible to decrease a possibility that regions of the internal surface of the balloon adhere to each other to decrease a possibility that the balloon is not loosened by the adhesion of regions of the internal surface.

The balloon for IABP balloon catheter according to the present invention may be made of polyurethane. Polyurethane is a material best for catheter balloons; however, smooth surfaces of polyurethane easily adhere to each other. Thus, this structure is effective when this material is made to have a rough surface.

In the IABP balloon catheter of the present invention, the above-mentioned balloon for IABP balloon catheter is adopted. As a result, the present invention can provide an IABP balloon catheter that has the above-mentioned advantageous effects.

### ADVANTAGEOUS EFFECTS OF INVENTION

According to the balloon for IABP balloon catheter according to the present invention, the balloon can be certainly unwrapped by a predetermined balloon pressure from a driving machine without the need to perform any preloading process. Additionally, the present invention can provide a balloon for IABP balloon catheter that is not unwrapped before and during the insertion of the catheter even in the state that a wrapping sheath is removed; and can provide a balloon catheter with the balloon having these advantageous effects.

### BRIEF DESCRIPTION OF DRAWINGS

Fig. 1 is a view which schematically illustrates the structure of an IABP balloon catheter 100, and that of a balloon 10 for IABP balloon catheter according to an embodiment.
Figs. 2A and 2B are each a view which illustrates a modified example of rough surfaces of the balloon 10 for IABP balloon catheter according to the embodiment.
Figs. 3A and 3B are each a view which illustrates a modified example of the rough surfaces of the balloon 10 for IABP balloon catheter according to the embodiment.
Figs. 4A and 4B are each a view which illustrates a modified example of the rough surfaces of the balloon 10 for IABP balloon catheter according to the embodiment.
Figs. 5A and 5B are each a view which schematically illustrates the structure of the IABP balloon catheter 100, and that of the balloon 10 for IABP balloon catheter according to the embodiment.
Figs. 6A and 6B are each a view which schematically illustrates a method of using the IABP balloon catheter 100 according to the embodiment.
Fig. 7 is a view which schematically illustrates the method of using the IABP balloon catheter 100 according the embodiment.
Fig. 8 is a view which illustrates the balloon 10 in the state of being unfavorably unwrapped.
Fig. 9 is a view which illustrates an inconvenience of a conventional balloon catheter 1.

### DESCRIPTION OF EMBODIMENT

### (Embodiment)

Hereinafter, with reference to the drawings, a detailed description will be given of a balloon 10 for IABP balloon catheter according to an embodiment, and an IABP balloon catheter 100 that has this balloon 10.

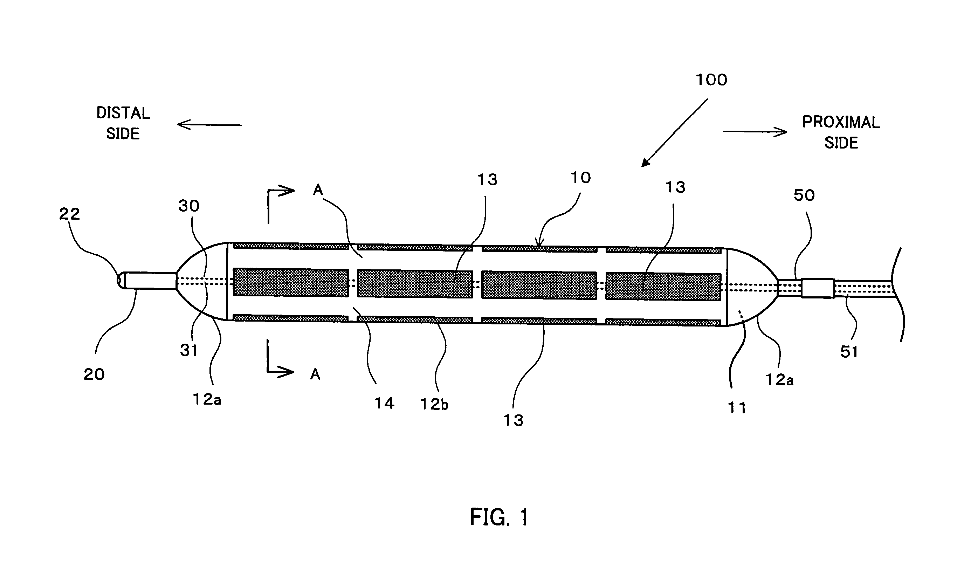

Fig. 1 is a schematic view illustrating the distal side of the IABP balloon catheter 100 with which the balloon 10 for IABP balloon catheter according to the embodiment is fitted. The IABP balloon catheter 100 according to the embodiment has a tip part 20 fitted with the distal side end of the IABP balloon catheter 100, an inner tube 30 having a guide wire lumen 31 connected to this tip part 20, the balloon 10 located around this guide wire lumen 31, and an outer tube 50 having an air-injecting lumen 51 connected to a balloon lumen 11 of this balloon 10. The balloon 10 is inflated by air that has passed through this air-injecting lumen 51.

The balloon 10 for IABP balloon catheter according to the present embodiment is made of polyurethane in the form of a thin membrane having a thickness of 40 to 150 µm. Its distal side end and its proximal side end each have a conic part 12a made into a substantially conic form. Its central region between these conic parts 12a is made into the form of a balloon having a columnar shape 12b. The columnar shape 12b referred to herein does not necessarily denote the shape of only a complete column. Thus, the center thereof may be swelled to some degree. As to the distal side end and the proximal side end of the balloon 10, their parts other than the conic parts 12a, which are each formed in the substantially conic form, have rough surfaces 13 which are crepe surfaces, and a smooth surface 14 made into a smooth form. It is conceivable that the method for forming the rough surfaces is a method of bonding a sheet subjected to crepe treatment onto the original surface, or a method of using a mold having a crepe surface to transfer a crepe pattern thereto. However, the method for the formation is not particularly limited.

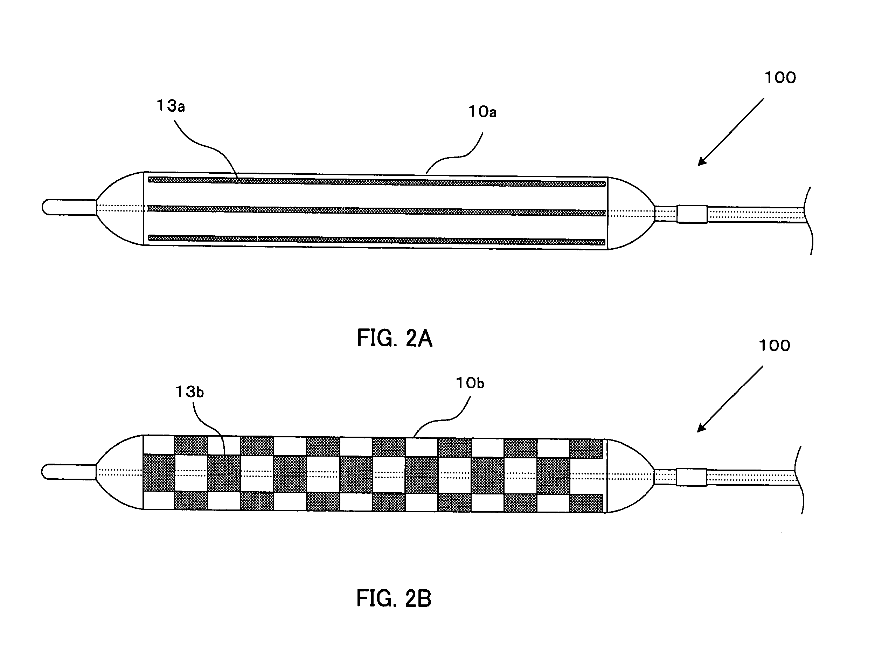

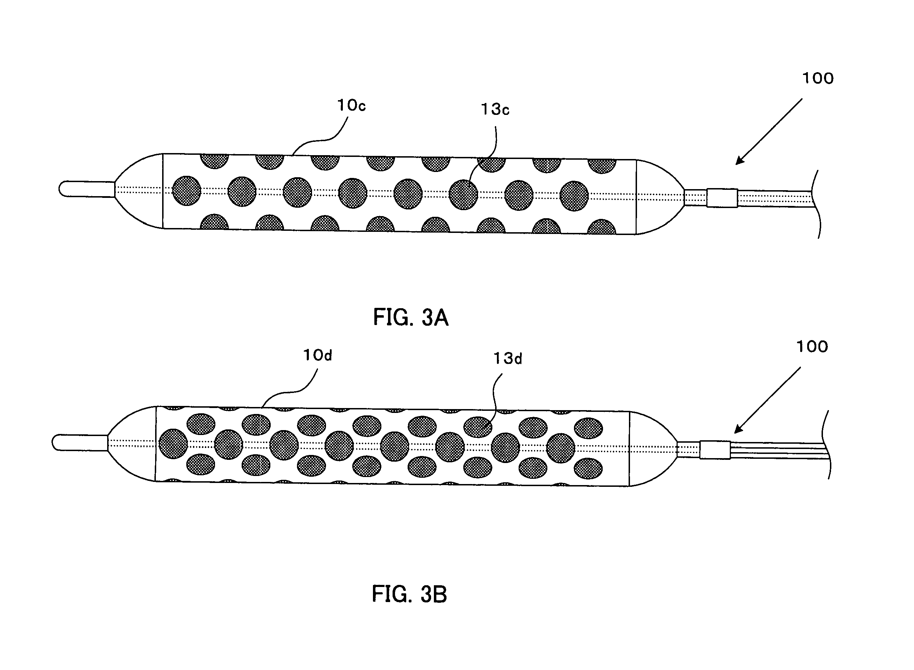

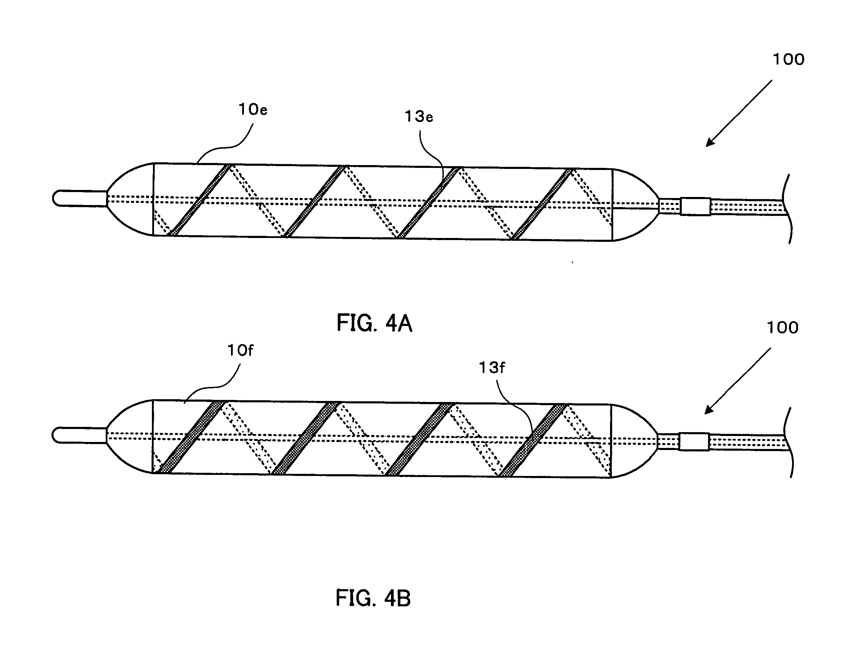

In the present embodiment, the rough surfaces 13 are formed in such a manner that crepe rectangles are dispersed to be arranged regularly in the circumferential direction and the longitudinal direction. The proportion by area of the rough surfaces 13 into the total external surface is 43.3%. The form of the rough surfaces and the manner of arranging the surfaces are not limited to the above. The shape of the rough surfaces may be any shape and the surface-arranging manner is not particularly limited as far as the proportion by area of the rough surfaces into the total external surface is from 10 to 80% both inclusive. The proportion by area is preferably from 10 to 50% both inclusive, more preferably from 20 to 40% both inclusive. Hereinafter, examples of the balloon that have various rough surfaces will be demonstrated. Fig. 2A illustrates a balloon 10a having rough surfaces 13a in the form of strips formed along the longitudinal direction of the balloon 10a and in parallel to each other. The proportion of the rough surfaces 13a into the total external surface is 19.6%. Fig. 2 B illustrates a balloon 10b having rough surfaces 13b formed in the external surface of the balloon 10b to be into a lattice form. The proportion of the rough surfaces 13b into the total external surface is 37.1%. Fig. 3A illustrates a balloon 10c in which circular rough surfaces 13c are located. The proportion of the rough surfaces 13c in Fig. 3A into the total external surface is 14.4%. Fig. 3B illustrates a balloon 10d in which circular rough surfaces 13d are located. The proportion of the rough surfaces 13d in Fig. 3B into the total external surface is 29.0%. Fig. 4A illustrates a balloon 10e in which a rough surface 13e is spirally located. The proportion of the rough surface 13e in Fig. 4A into the total external surface is 7.4%. Fig. 4B illustrates a balloon 10f in which a rough surface 13f is spirally located. The proportion of the rough surface 13f in Fig. 4B into the total external surface is 12.3%.

As illustrated in Fig. 1, the tip part 20 is fitted with the front end of the IABP balloon catheter 100 to close the front end side of the balloon 10, so that no fluid can flow out from the front end side of the balloon 10. At the center of the tip part 20, a through hole 22 which is connected to the guide wire lumen 31 is provided. This through hole 22 is a hole through which a guide wire (not illustrated) is to be passed when the guide wire is used to guide the balloon 10 to a detention position therefor. When this through hole is filled with physiological saline to be connected to a pressure sensor of a driving machine, the through hole is also used to monitor the internal pressure of an artery.

The guide wire lumen 31 is formed at the center of the inner tube 30 along the longitudinal direction thereof. The proximal side of the inner tube 30 is extended to the operator's hand, and the guide wire can be inserted from the proximal side end of the inner tube 30 thereinto.

The outer tube 50 has, at the center along the longitudinal direction thereof, the air-injecting lumen 51. Through this air-injecting lumen 51, the inner tube 30 is also being passed. The gap between the air-injecting lumen 51 and the inner tube 30 is connected to the balloon lumen 11.

The IABP balloon catheter 100 is wound onto the inner tube 30. In this state, the IABP balloon catheter is wrapped with a wrapping sheath 60 to complete the balloon catheter.

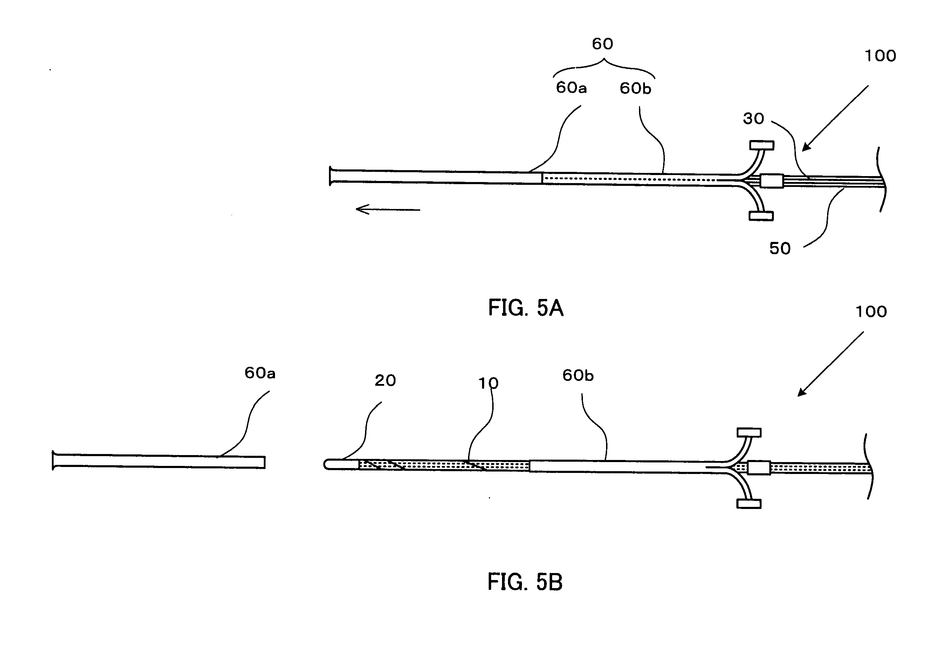

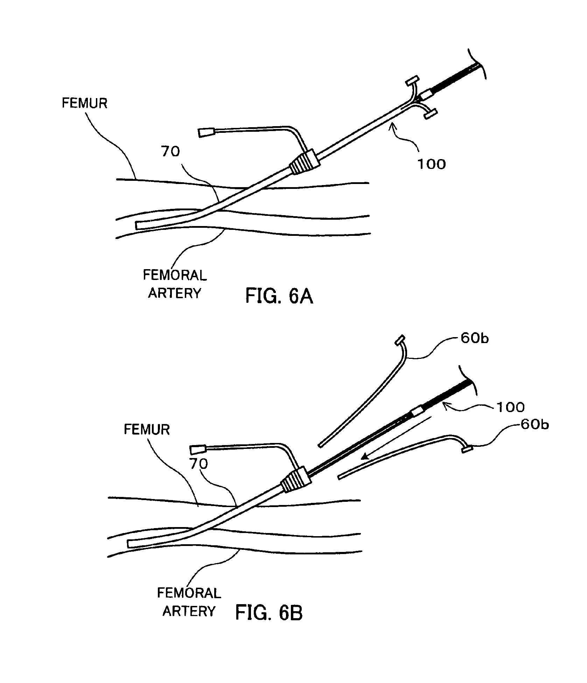

The following will describe a method of using the thus produced balloon 10, and the IABP balloon catheter 100, giving, as an example, a state that the balloon catheter 100 is inserted into a femoral artery and then the balloon is detained in the descending aorta. Before the IABP balloon catheter 100 is used, as illustrated in Fig. 5A, the balloon catheter 100 is wrapped with the wrapping sheath 60 in the state that the balloon 10 is wound on the inner tube 30. As illustrated in Fig. 5B, from this pre-use state, the balloon catheter 100 is made into a state that a first wrapping sheath 60a is removed to make substantially half the balloon 10 naked. In this state, the balloon 10 is inserted, by half, into a sheath 70 inserted separately into the femoral artery (Fig. 6A). Thereafter, as illustrated in Fig. 6B, a second wrapping sheath 60b (of the sheath 60) is torn away to make the balloon 10 into a completely naked state. In this state, the balloon catheter is slowly inserted into the femoral artery.

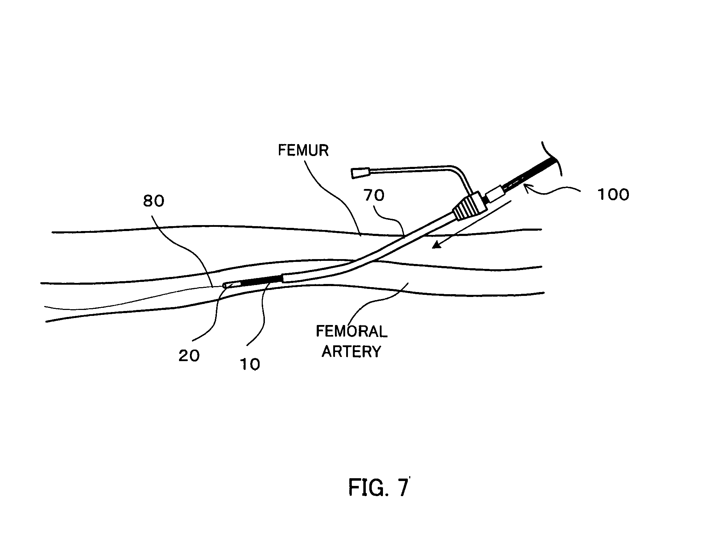

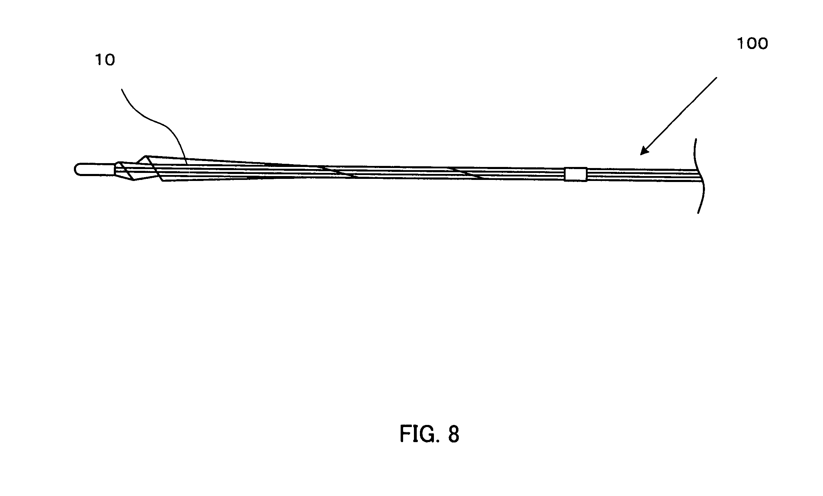

As illustrated in Fig. 7, thereafter, a guide wire 80 is inserted from the tip part 20 of the balloon 10 to a detention position of the descending aorta. The guide wire 80 is used to guide the balloon 10 to the detention position and then to be detained in the detention position. If the wrap-adhesion in the wrapping state of the balloon 10 is weak, the wrapping for the balloon 10 may be loosened as illustrated in Fig. 8. When the wrapping is loosened, it may cause a problem that inserting the IABP balloon catheter 100 is difficult or a problem that moving the balloon catheter 100 inside the blood vessel during the insertion is difficult. However, the balloon 10 according to the embodiment has, in the external surface thereof, the smooth surface 14 to an appropriate degree. Thus, regions of the smooth surface 14 are caused to adhere to each other so that the balloon 10 can be prevented from being unwrapped when the balloon 10 is operated.

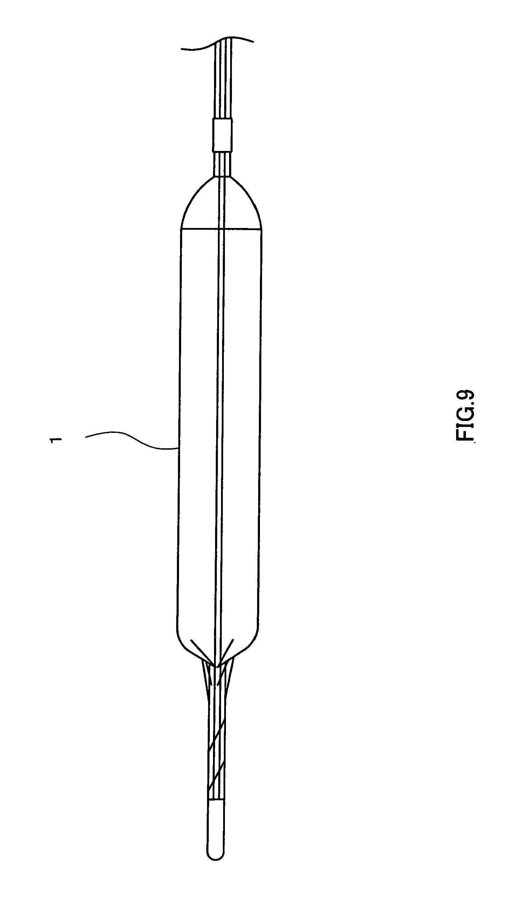

In this way, the balloon 10 is detained in the detention position, and subsequently the balloon 10 is inflated. As illustrated in Fig. 9, in the case of using a balloon catheter 1 in the prior art, the balloon catheter 1 may not be partially unwrapped when its polyurethane regions adhere to each other by tight wrapping. Thus, the operator needs to use a syringe to perform a preloading process for completely expanding the balloon one time, thereby attaining the complete expanding once. After the complete expanding, a driving machine is fitted with the catheter. However, in the present embodiment, in the external surface of the balloon 10, the rough surfaces are formed; the same complete expanding can be attained by the blowing pressure of air from the driving machine for air-blowing. It is therefore possible to fit the driving machine for air-blowing, from the proximal side of the IABP balloon catheter 100, directly to the balloon catheter 100, and then blow air to the balloon 10 to attain the complete expanding easily without performing any preloading process.

The present invention is not limited to each of the above-mentioned embodiments. It is needless to say that the present invention can be carried out in the form of various embodiments as far as the embodiments belong to the technical scope of the present invention.

In the above-mentioned embodiment, polyurethane is used as the raw material of the balloon 10. However, the material is not limited thereto. Silicone, latex, or any different resin is usable as far as the resin has an adhesive property.

In the above-mentioned embodiment, crepe surfaces are used as the rough surfaces. However, grain surfaces may be used, or a plurality of small convex projections may be formed on the surfaces.

In the above-mentioned embodiment, various examples of the form of the rough surfaces have been described. However, the form is not limited thereto. Thus, the rough surfaces may be rough surfaces in any form, and the arranging manner thereof is not particularly limited as far as the proportion by area of the rough surfaces into the total external surface is from 10 to 80% both inclusive.

In the above-mentioned embodiment, the rough surfaces are formed in the external surface of the balloon 10 for IABP balloon catheter. However, the same rough surfaces may be formed in the internal surface of a balloon for IABP balloon catheter. This manner makes it possible to decrease the possibility that regions of the internal surface of the balloon for IABP balloon catheter adhere to each other, so as to decrease the possibility that the balloon for IABP balloon catheter is not unwrapped by adhesion of the internal surface regions. The rough surfaces located in the internal surface may be made into a pattern form, as has been described about the above-mentioned embodiment. The rough surfaces located in the internal surface may be different from those located in the external surface.

### (Example)

Prepared were balloon membranes (made of polyurethane) each having the same rough surfaces as used in the balloon 10 for IABP balloon catheter, and balloon membranes (made of polyurethane) each having the same smooth surfaces as used in the balloon 10. Adhesion was made between mirror surfaces, between a mirror surface and a rough surface, and between rough surfaces. The resultants were each heated at 55°C for 48 hours while a load (0.5 kg/cm3 or more) was applied thereto. In this way, the membranes were caused to adhere to each other. The membrane-adhered products were each cut into dumbbell test pieces (No. 3) in JIS K 6251. A tensile tester was used to measure T-peel strength and shear-peel strength of each of the test pieces at a tensile speed of 20 mm/min. The test pieces in which the adhesion of the rough surfaces to each other was attempted, were not adhered. Table 1 shows results of the T-peel test; and Table 2, results of the shear-peel test.

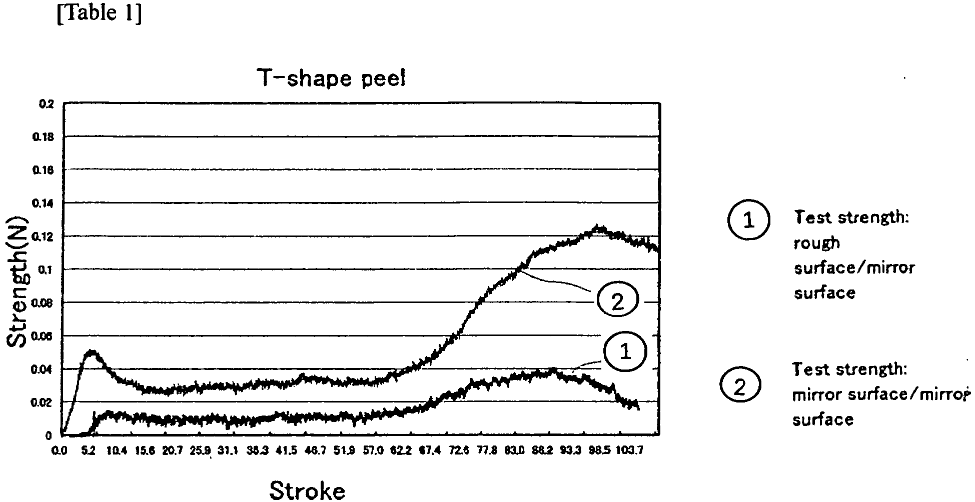

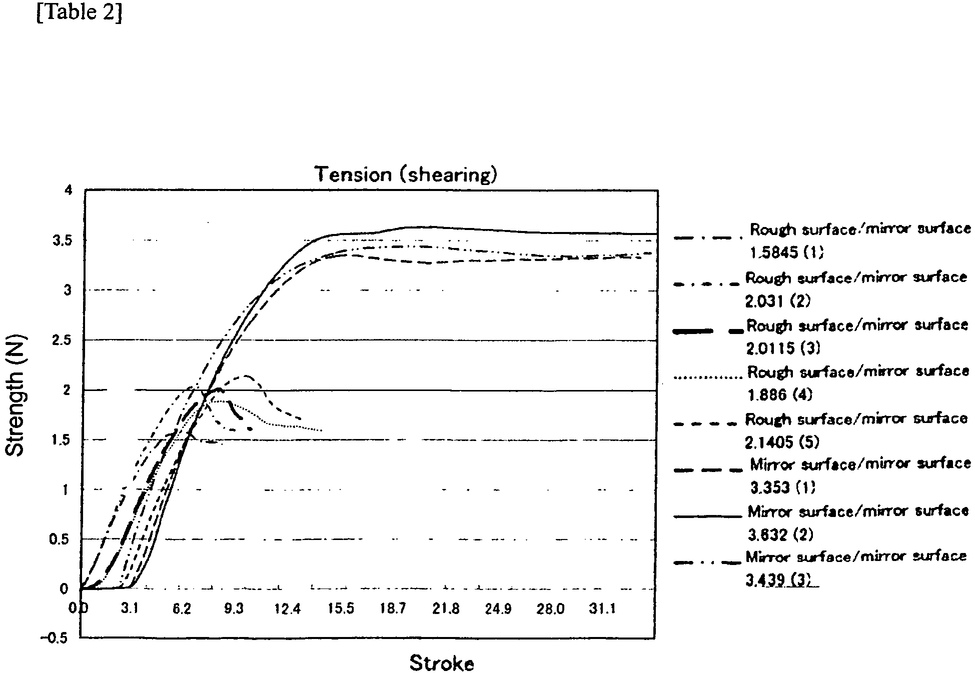

According to Table 1, the T-peel strength of the mirror-surface/mirror-surface test pieces was about 0.03 N while that of the mirror-surface/rough-surface test pieces was about 0.01 N. It has been understood that the rough-surface/mirror-surface combined test pieces are smaller in peel strength than the mirror-surface/mirror-surface test pieces but no peeling occurred unless 0.01 N is applied. As shown in Table 2, the following has been understood about the shear-peel strength: in the mirror-surface/mirror-surface test pieces, a strength of 3.4 to 3.7 N is required for a peel therein; and in the rough-surface/mirror-surface combined test pieces, a strength of about 1.5 to 2.1 N is required for a peel therein. In the case of preparing a balloon surface having a peel strength in such a range, that is, using a rough surface and a mirror surface, rendering the rough surface a rough surface occupying an area of 10 to 80% both inclusive of the whole of the external surface, and rendering the mirror surface a smooth surface (mirror surface) occupying an area of 90 to 20% both inclusive of the whole, a balloon for IABP balloon catheter can be provided which is certainly unwrapped by a predetermined balloon pressure from a driving machine, and further which is not unwrapped before and during the insertion of the catheter even in the state that a wrapping sheath is removed. Furthermore, a balloon catheter with the balloon having the advantageous effects can be provided.

### INDUSTRIAL APPLICABILITY

As has been described about the above-mentioned embodiment, the present invention is usable for an IABP balloon catheter that is used for an auxiliary circulation when a fall in cardiac function, such as cardiac insufficiency, is caused, and is usable as its balloon.

### LIST OF REFERENCE SIGNS

10: balloon for IABP balloon catheter, 11: balloon lumen, 12: conic part, 13: rough surface, 14: smooth surface, 20: tip part, 22: through hole, 30: inner tube, 31: guide wire lumen, 50: outer tube, 51: air-injecting lumen, 60: wrapping sheath, 60a: first wrapping sheath, 60b: second wrapping sheath, 70: sheath, 80: guide wire, and 100: IABP balloon catheter.

## Claims

1. A balloon for IABP balloon catheter, the balloon being fitted with a medical catheter, wherein:
the external surface of the balloon for IABP balloon catheter has a rough surface occupying an area of 10 to 80% both inclusive of the whole of the external surface, and a smooth surface occupying an area of 90 to 20% both inclusive of the whole of the external surface.

2. The balloon for IABP balloon catheter according to claim 1, wherein the rough surface is dispersed in at least the whole of a location other than a substantially conic part located at the distal side or proximal side of the balloon for IABP balloon catheter.

3. The balloon for IABP balloon catheter according to claim 1 or 2, wherein the rough surface is formed only in a location other than a substantially conic part formed at the distal side or proximal side of the balloon for IABP balloon catheter.

4. The balloon for IABP balloon catheter according to any one of claims 1 and 2, wherein the rough surface is a crepe surface, or a grain surface.

5. The balloon for IABP balloon catheter according to any one of claims 1 and 2, wherein the rough surface is made into any one of striped, checkered, dotted, and spiral patterns, or into a pattern of a combination thereof.

6. The balloon for IABP balloon catheter according to any one of claims 1 to 5, wherein the rough surface is formed in at least one part of the internal surface of the balloon for IABP balloon catheter.
